# EUROPÄISCHE PATENTSCHRIFT

(11) **EP 0 681 462 B1**
(45) Veröffentlichungstag und Bekanntmachung des Hinweises auf die Patenterteilung: **09.04.1997**
(21) Anmeldenummer: 94906183.2
(22) Anmeldetag: 31.01.1994
(51) Int. Cl.: A61C 5/02, A61C 3/03, A61C 8/00

(54) **INSTRUMENTE ZUR ZAHNBEHANDLUNG**
DENTAL TREATMENT INSTRUMENTS
INSTRUMENTS POUR SOINS DENTAIRES

(30) Priorität: 29.01.1993 CH 263/93
(43) Veröffentlichungstag der Anmeldung: 15.11.1995
(73) Patentinhaber: Ilgenstein, Bernd, Dr., 4515 Oberdorf (CH)
(72) Erfinder: Ilgenstein, Bernd, Dr., 4515 Oberdorf (CH)
(74) Vertreter: Kaminski, Susanne, Dr.
(86) Internationale Anmeldenummer: EP9400271
(87) Internationale Veröffentlichungsnummer: WO9416640

(56) Entgegenhaltungen:
- EP-A- 0 074 331
- EP-A- 0 373 111
- WO-A-93/07819
- DE-B- 1 294 592
- DE-C- 758 727
- FR-A- 723 763
- FR-A- 2 566 262
- GB-A- 2 228 872
- US-A- 2 874 470
- US-A- 4 109 384
- US-A- 4 340 364
- US-A- 4 521 191

## Beschreibung

Die Erfindung betrifft ein Zahnbehandlungsinstrument zur retrograden Behandlung von Wurzelkanälen, nach dem Oberbegriff des Anspruchs 1 und ein Set von Zahnbehandlungsinstrumenten.

Die im Zahninneren gelegene Pulpahöhle ist meist frühzeitig an dem den Zahn befallenden Infektionsprozess beteiligt. Von Bakterien gebildete Toxine dringen in die Pulpa vor und lösen dort entzündliche Reaktionen aus. Wird der Zahn nicht rechtzeitig behandelt, können sich die Bakterien in der Pulpa ansiedeln und die daraus resultierende, eitrige Entzündung kann sich auf die Zahnwurzel und den angrenzenden Knochen ausdehnen. In einem solchen Fall muss das Zahnmark entfernt und der Wurzelkanal mit einem abdichtenden Material gefüllt werden, eine als Wurzelfüllung oder Wurzelkanal-Therapie bezeichnete Methode.

Der Behandlung der Zahnwurzelkanäle von der Zahnkrone her mittels bekannter, insbesondere rotierender, Aufbereitungsinstrumente ist aber aufgrund der teils nicht unbeträchtlichen Krümmungen und der Querschnittsform der Kanäle gewisse Grenzen gesetzt.

Eine Art dieser Behandlung wird und muss von der Wurzelspitze her vorgenommen. Dazu wird durch einen Schnitt im Bereich der Schleimhaut der die Wurzelspitze umgebende Knochen freigelegt und in bekannter Weise abgetragen. Rosenbohrer dienen der Präparation des Knochens zur Darstellung der Wurzelspitze; mittels eines Finierers ohne Quertrieb wird die Wurzelspitze abgetrennt. Aus Gründen der Sterilität sollten Rosenbohrer und Finierer aus Stahl sein.

Die bei dieser Art von Wurzelbehandlung auftretenden Probleme beruhen auf den sehr eingeschränkten Platzverhältnissen und oft auf einer nur seitlichen Zugangsmöglichkeit.

Die direkte, seitliche Bearbeitung der Wurzelspitze wird jedoch mittels der bekannten, rotierenden Behandlungsinstrumente handhabungsmässig schwierig, ebenso wie die achsengerechte Aufbereitung des Wurzelkanallumens. Auch sollten die Instrumente sowohl unter dem Gesichtspunkt der bequemen Handhabung als auch unter dem Gesichtspunkt, nicht unnötig Knochensubstanz zu opfern, möglichst fein ausgebildet sein.

In der DE-A1-38 22 889 werden Zahninnenbehandlungs-Instrumente beschrieben, die an Griffen befestigbar sind, welche Schall- oder Ultraschallvibrationen übertragen. Die dort zum Einsatz kommenden Instrumente sind allerdings für Wurzelbehandlungen von der Spitze her ungeeignet.

Das in der US-A-5,094,617 beschriebene Behandlungsinstrument ist für die retrograde Präparation der Zahnwurzel vorgesehen. Dazu wird eine speziell geformte Bohrspitze in Ultraschallschwingungen versetzt. Die Spitze selbst besteht aus einem länglichen Schaftabschnitt, der eine Freistellbiegung aufweist, sowie aus einem zu der durch die Freistellbiegung definierten Ebene winkelig versetzt angeordneten, hakenförmigen Teil. Ein solches Instrument besitzt gegenüber den klassischen, für die retrograde Aufbereitung verwendeten Instrumenten nicht unbeträchtliche Vorteile, da insbesondere die Zugänglichkeit zum Wurzelkanal verbessert und somit die Grösse des abzuhebenden Knochendeckels reduziert werden kann. Ausserdem wird eine dem tatsächlichen Wurzelkanalverlauf entsprechende Präparation möglich.

Wird in einen mit einer solchen Bohrspitze präparierten Wurzelkanal Füllmasse eingebracht, so kann diese nicht in ausreichender Weise "verankert" werden.

Die Behandlung von Wurzelkanälen ist allerdings nur dann als erfolgreich zu bezeichnen, wenn nach erfolgter Entfernung der Entzündungsherde der präparierte Wurzelkanal durch geeignetes Füllmaterial hermetisch und bakteriendicht abgeschlossen werden kann, so dass weder vom Restlumen der Pulpa noch durch eine Lockerung der retrograden Zahnfüllung eine Insuffizienz befürchtet werden muss. Aus der US-A-5,094,617 ist allerdings weder ein Hinweis auf das Problem selbst noch auf eine mögliche Lösung desselben zu entnehmen. Sind solche Überlegungen zwar auch für die Behandlung von Dentinkaries wichtig, so stellt sich allein aufgrund der vergleichweise guten Zugänglichkeit bei der Präparation von Dentinkavitäten und der relativ einfachen Überwachbarkeit von Inlays dieses Problem dabei nicht in dieser Form.

Die Erfindung hat sich demgegenüber die Aufgabe gestellt, Instrumente bereitzustellen, mit deren Hilfe die retrograde Präparation von Wurzelkanälen bzw. das - ebenfalls retrograde - Einbringen von Wurzelfüllmaterial in die präparierten Wurzelkanäle möglich wird, und das sicher und einwandfrei trotz der beschränkten Platzverhältnisse und der schwierigen Zugänglichkeit zu den Wurzelspitzen. Das gelingt durch die Verwirklichung der kennzeichnenden Merkmale des Anspruchs 1.

Vorteilhafte Weiterbildungen werden durch die Merkmale der abhängigen Ansprüche beschrieben.

Ist - im Querschnitt gesehen - der Durchmesser des Kopfes senkrecht zur Längserstreckung der Spitze gemessen grösser als der Durchmesser des Kopfes in Richtung der Längserstreckung der Spitze gemessen, der Kopf also beispielsweise wulst-, linsen- oder auch birnenförmig ausgebildet, so eignet sich ein solches Zahnbehandlungsinstrument sowohl als Aufbereitungsinstrument zur Präparation des Wurzelkanals, so dass dieser etwas erweitert wird und gleichzeitig Hinterschneidungen gebildet werden, als auch als Einbringinstrument, mittels dem in den präparierten Wurzelkanal eingebrachtes Wurzelfüllmaterial komprimiert und sicher an der Wurzelkanalwandung festgedrückt wird. Insbesondere aufgrund der hinterschnitten ausgebildeten Erweiterung des Wurzelkanals wird darin eingebrachtes Wurzelfüllmaterial sicher im Wurzelkanal gehalten.

An der Spitze vorgesehene Markierungen erlauben die Kontrolle sowohl über die Eindringtiefe des Instrumentes, als auch über dessen Geradführung, eine insbesondere für das korrekte Aufbereiten des Wurzelkanals unerlässliche Forderung.

Freistellbiegungen am Schaft der Zahnbehandlungsinstrumente, nach rechts oder nach links weisend, ermöglichen eine bequeme und ungehinderte Behandlung in den verschiedenen Quadranten. Zahnbehandlungsinstrumente mit nach rechts bzw. nach links weisenden Freistellbiegungen entsprechen damit spiegelsymmetrischen Varianten. Derartige Zahnbehandlungsinstrumente werden insbesondere zur Behandlung der Backenzähne verwendet, während Behandlungsinstrumente ohne Freistellbiegung für die Behandlung der Schneidezähne verwendet werden können.

Zum Erzielen einer einwandfreien Präparation des Wurzelkanals mittels eines Instrumentes, wie es in der US-A-5,094,617 beschrieben ist, sind hohe Schwingungsfrequenzen erforderlich. Nur dann wird Zahnmaterial abgetragen. Bei derart hohen Frequenzen wird der in den Wurzelkanal eingeführte hakenförmige, im wesentlichen transversal schwingende Teil des dort geoffenbarten Zahnbehandlungsinstrumentes sich erhitzen. Dies kann durch Weiterleitung der zu hohen Temperaturen - da es bei Temperaturen von über 53° C zur beginnenden Denaturierung kommt - zu Knochenschäden im umgebenden Kieferknochen führen. An sich gesunde Zahnsubstanz verbrennt. Zudem neigt eine derart erhitzte, an sich sehr dünne Spitze dazu, abzubrechen, ein sicherlich äusserst unerwünschter Tatbestand.

Wird hingegen der Bohrkopf eines erfindungsgemässen Aufbereitungsinstrumentes diamantiert, so kann auch bei niedrigeren Frequenzen gearbeitet werden und doch sicher Zahnmark und/oder Zahnbein entfernt werden. So kann auch bei relativ niedrigen (Schall-) Frequenzen von beispielsweise 6500 Hertz (hier haben sich für die Diamant-Belegung Korngrössen zwischen 100 und 180 µm als praktikabel gezeigt) gearbeitet werden, womit die oben beschriebenen Nachteile behebbar sind. Es ist offensichtlich, dass unabhängig von der Ausbildung des Kopfes eine solche Diamantbelegung zweckmässig ist. So ist eine solche Diamantbelegung auch für Bohrköpfe von Vorteil, die beispielsweise zylinderförmig oder konisch - insbesondere mit gebrochen geformter Randkante - ausgebildet sind und die insbesondere für die Vorpräparation des Wurzelkanals geeignet sind. Die Wahl der Korngrösse der Diamantierung wird dementsprechend in Abhängigkeit von der Frequenz, mit der das Aufbereitungsinstrument schwingt, zu wählen sein. Je höher die Frequenz, desto geringer kann die durchschnittliche Korngrösse der Diamantbelegung gewählt werden.

Die Kühlung der schwingenden Spitze eines Aufbereitungsinstrumentes wird insbesondere dann in vorteilhafter Weise vorzusehen sein, wenn diese mit relativ hohen Frequenzen schwingt, um der Erwärmung derselben bzw. des behandelten Zahnbeins und gegebenenfalls des umgebenden Knochenmaterials entgegen zu wirken. Zwar wäre ein bis an den Bohrkopf reichender, im Inneren des Schaftes und vorzugsweise auch im Inneren der Spitze angeordneter, zur Aufnahme von Kühlflüssigkeit vorgesehener Kanal besonders zweckmässig, jedoch wird sich diese Anordnung bei den für die meisten Zahnwurzelbehandlungen zu verwendenden, im allgemeinen sehr dünnen Spitzen nicht empfehlen, da dies eine nicht zu vernachlässigende Schwächung der Spitze bedingen könnte. Nach dem auch durch Wahl geeigneter, hochfester Materialien entgegengewirkt werden können, so wird sich doch - insbesondere für sehr dünne Spitzen-Durchmesser - eine Anordnung des Kanals an der Aussenseite des Schaftes und gegebenenfalls auch an der Aussenseite der Spitze als zweckmässiger erweisen.

Da die Spitze, die ja gegenüber dem im wesentlichen in longitudinale Schwingungen versetzten Schaft des Aufbereitungsinstrumentes im wesentlichen transversal schwingt, ist eine solche Ausbildung des die Kühlflüssigkeit führenden Kanals zu bevorzugen, bei der dieser wenigstens noch teilweise an der Spitze entlanggeführt ist, bzw. wenigstens bis zur Knickstelle Schaft-Spitze reicht, dann insbesondere spiralförmig verlaufend, damit die austretende Kühlflüssigkeit nicht abgespritzt wird, sondern bis zum Bohrkopf und damit bis an die Behandlungsstelle in der Zahnwurzel "kriechen" kann.

Die erfindungsgemässen Aufbereitungsinstrumente werden - da nicht rotationssymmetrisch - durch Schall- bzw. Ultraschallgeber in Schwingungen versetzt, vorzugsweise durch Einstecken in dazu vorgesehene und geeignete Griffe.

Erfindungsgemässe Einbringinstrumente können vorteilhafterweise, da reine Handinstrumente, zwei an den beiden Enden eines Handgriffes unterschiedlich ausgebildete Schaftenden aufweisen, entweder zwei zueinander spiegelsymmetrisch ausgebildete Enden mit jeweils gleich geformter Spitze, oder auch zwei, für aufeinanderfolgende Einbringschritte unterschiedlich ausgebildete Spitzenformen, wobei die gegebenenfalls vorgesehene Freistellungsbiegung in jeweils die gleiche Richtung weist, für die Arbeit in demselben Quadranten.

Der Ablauf der Behandlung wird erleichtert, wenn dem behandelnden Arzt ein Set von Zahnbehandlungsinstrumenten zur Verfügung steht, das in abgestimmter Weise die speziellen Aufbereitungsinstrumente und Einbringinstrumente beinhaltet.

Da im allgemeinen die Präparierung des Wurzelkanals ebenso wie die Füllung des präparierten Kanals in zwei aufeinanderfolgenden Schritten erfolgt, wie oben erwähnt, sind in einem solchen Set vorzugsweise wenigstens zwei Aufbereitungsinstrumente unterschiedlicher Kopfform und wenigstens zwei Einbringinstrumente mit Kopfformen, die denjenigen der Aufbereitungsinstrumenten entsprechen, untergebracht.

In dem Set sollten Aufbereitungs- und Einbringinstrumente mit unterschiedlichen Kopfdurchmessern zur Verfügung stehen, entsprechend den ISO-Normen (International Standardization Organization). So sind Instrumente unterschiedlicher ISO-Grössen je nach zu behandelndem Zahn erwünscht und auch erforderlich, um die notwendige Grösse des aufzubereitenden Wurzelkanals zu erreichen und gleichzeitig die restliche laterale Zahnsubstanz nicht zu schwächen. Wenigstens jeweils zwei gleiche Zahnbehandlungsinstrumente mit unterschiedlichem Durchmesser des Kopfes sollten in diesem Set vorgesehen sein, mit gegebenenfalls gestaffelten Durchmesserwerten, die beispielsweise zwischen 0,3 mm und 10 mm liegen, der ISO-Norm 30 bzw. 100 entsprechend.

Vorzugsweise ist einem solchen Set auch der die Schall-bzw. Ultraschallschwingungen erzeugende bzw. übertragende Griff beigeordnet, in den die benötigten Aufbereitungsinstrumente eingesteckt werden können.

Da, wie einleitend dargestellt, für die Darstellung der Wurzelspitze der Kieferknochen präpariert werden muss, entsteht möglicherweise ein grosser Verlust an Knochensubstanz. Um dies zu vermeiden, werden Knochenaufbereitungsinstrumente verwendet, mit deren Hilfe Knochendeckel gewünschten Durchmessers ausgefräst werden. Zweckmässigerweise werden derartige Knochenaufbereitungsinstrumente dem Set beigefügt.

Bekannte Knochenaufbereitungsinstrumente - als Trepanationsbohrer bezeichnet - fräsen einen kreisförmigen Deckelbereich aus, der, da die Verbindung zu den tieferen und weicheren Knochenbezirken (Spongiosa), die die Zahnwurzel umgeben, zumindest bei gesundem Knochenmaterial relativ fest ist, meistens abgestemmt werden muss. Dabei kann es zum Absplittern von Knochen kommen, was nach dem Wiedereinsetzen des Knochendeckeis nach erfolgter Wurzelbehandlung gegebenenfalls zu verlangsamtem oder fehlendem Wiedereinwachsen des Knochendeckels in den Kiefer führt, oder auch daraus resultierend einen möglichen Herd für Knochenentzündungen ergibt.

Um eine Erhitzung des Knochens zu vermeiden, sollten Trepanationsbohrer mit einer - insbesondere Kühlflüssigkeit zur Verfügung stellenden - Kühleinrichtung ausgestattet sein.

Ein Knochendeckelaufbereitungsinstrument, dem ein Retentions- und/oder Repositionsinstrument zugeordnet ist, zeichnet sich dadurch aus, dass nach Ausfräsen des Knochendeckels dieser mit dem Retentionsinstrument entfernt werden kann, ohne dass ein die Schnittkanten des Knochendeckels bzw. des umgebenden Knochens in Mitleidenschaft ziehendes Abstemmen erfolgen muss. Über ein Repositionsinstrument, wobei vorzugsweise das Retentionsinstrument derart ausgebildet ist, dass es auch der Reposition dient, kann der Knochendeckel - und zwar positionsgenau - wieder in das Loch im Kieferknochen eingesetzt werden. Retentions- und/oder Repositionsinstrument können an sich als eigene Instrumente vorgesehen sein, werden allerdings zweckmässigerweise als Bestandteile des Knochendeckelaufbereitungsinstrumentes ausgebildet sein. Es ist offensichtlich, dass solche Retentions- und/oder Repositionsinstrumente, gegebenenfalls im Verbindung mit einem an sich bekannten Trepanationsbohrer, auch unabhängig von ihrer Anordnung im Set und ihrer Anwendung gemeinsam mit den anderen Zahnbehandlungsinstrumenten, die der retrograden Wurzelbehandlung dienen, von Interesse sind.

Das Retentions- bzw. Repositionsinstrument kann beispielsweise in Form eines Stabes mit sich durch - beispielsweiser manuell erfolgter - Drehbewegung an einem Schaft, vorzugsweise sternartig sich an der Spitze des Stabes entfaltenden Gebildes - und vorzugsweise im Zentrum des Trepanationsbohrers angeordnet - ausgebildet sein. Derartige Systeme sind beispielsweise in Carpulenspritzen zum Aspirieren gebräuchlich. Der Stab wird entweder in ein bereits vorgebohrtes Loch eingeführt wird, oder dient gegebenenfalls selbst als Bohrer. Auch als Fixation, Zentrierungshilfe oder gegebenenfalls Führung des Trepanationsbohrers kann es dienen. Mittels des Retentionselementes, das unter der Knochendecke ausgebracht wird, kann der Knochendeckel, nachdem er ausgefräst worden ist, abgenommen werden. Bei einer derartigen Ausbildung dient das Retentionselement in vorteilhafter Weise auch dazu, den Knochendeckel innerhalb des Hohlzylinders des Trepanationsbohrers festzuhalten. Nach erfolgter Wurzelbehandlung kann der Knochendeckel wieder eingesetzt werden, das Retentionselement wird zurückgezogen und, nachdem es an der äusseren Seite des Knochendeckels neuerlich ausgebracht wurde, als Repositionselement eingesetzt werden.

Sind an der Aussenseite des Hohlzylinders des Trepanationsbohrers Markierungen vorgesehen, so kann während des Fräsvorgangs die jeweilige Eindringtiefe - Millimeter-gradiert - kontrolliert werden. Mittels eines einstellbaren Anschlangsrings oder Anschlagszylinders, die insbesondere an der Aussenseite des Holzylinders angeordnet sind, kann sowohl die gewünschte Eindringtiefe eingestellt und fixiert werden, als auch das richtig beabstandete Wiedereinsetzen des Knochendeckels erfolgen.

Die Erfindung wird im folgenden anhand der Zeichnungen rein beispielhaft beschrieben. Es zeigen:
- Fig.1a bis 1d: die einzelnen Schritte einer Wurzelbehandlung bei Einsatz erfindungsgemässer Aufbereitungsinstrumente;
- Fig.2a bis 2d: erste Aufbereitungsinstrumente zum Entfernen des Wurzelkanalinhalts von oben und von der Seite gesehen, für die Behandlung in unterschiedlichen Quadranten;
- Fig.3a bis 3d: zweite Aufbereitungsinstrumente zur Erzeugung eines Unterschnitts im Wurzelkanal von oben und von der Seite gesehen, für die Behandlung in unterschiedlichen Quadranten;
- Fig.4a bis 4d: Einbringinstrumente zum Plazieren und Komprimieren von Wurzelfüllmaterial, von oben und von der Seite gesehen;
- Fig.5a bis 5e: eine Auswahl möglicher Ausbildungen für Kopfformen erfindungsgemässer Behandlungsinstrumente und
- Fig.6: ein Knochendeckelaufbereitungsinstrument mit Retentions- bzw. Repositionselement.

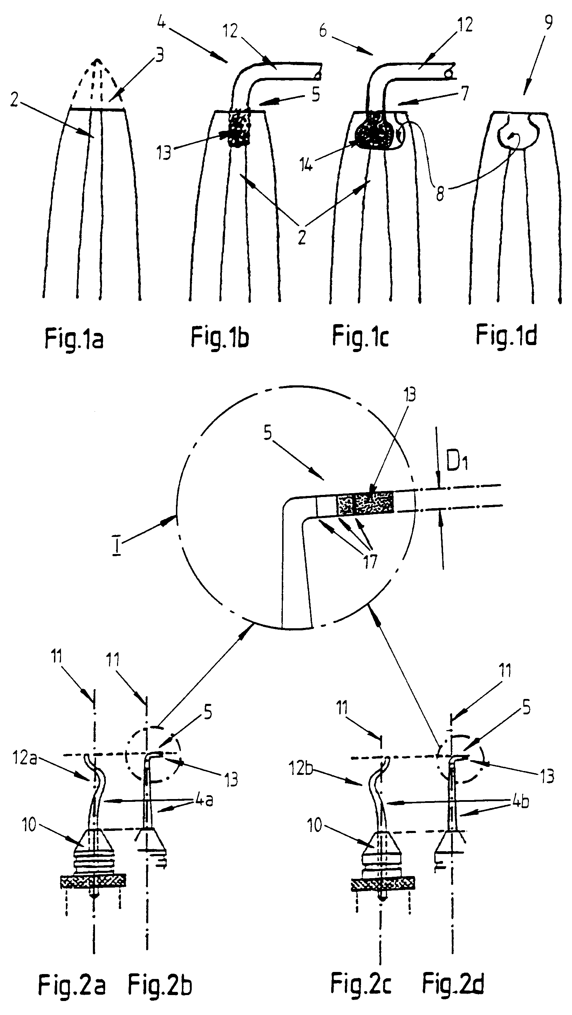

Die einzelnen Schritte einer Wurzelbehandlung, bei der erfindungsgemässe Instrumente zum Einsatz kommen, sind aus den Fig.1a bis 1d zu entnehmen. Nach einem Schnitt im Bereich der Schleimhaut wird die Wurzelspitze 3 dargestellt, wobei der Knochen mittels eines bekannten Rosenbohrers oder, in bevorzugter Weise, mittels eines - wie weiter unten beschrieben - Trepanationsbohrers präpariert wurde. Die Wurzelspitze 3 wird danach mit Hilfe eines bekannten Finierers ohne Querhieb entfernt, der Wurzelkanal 2 liegt somit frei (Fig.1a). Rosenbohrer und Finierer sind vorzugsweise aus Stahl geformt.

Ein in den Figuren 2a bis 2d näher beschriebenes, erstess Aufbereitungsinstrument 4 mit gegenüber einem Schaft 12 um ca 90° abgebogener Spitze 5 wird in den Wurzelkanal 2 eingebracht und über Schall oder Ultraschall in Schwingungen versetzt. Die Spitze 5 trägt einen zylinderförmig ausgebildeten Kopf 13, der, in Abhängigkeit von der verwendeten Schwingungsfrequenz, gegebenenfalls diamantiert ist. Der Rand des zylinderförmigen Kopfes 13 ist dabei vorzugsweise gebrochen geformt, wodurch aufgrund der leichten Konizität des Kopfes 13 das korrekte Einbringen des schwingenden Instrumentes vereinfacht ist. Mit Hilfe dieses ersten Aufbereitungsinstrumentes 4 wird der Inhalt des Wurzelkanals 2 entfernt. Sollte bei diesem ersten Präparationsschritt eine Abtragung von Zahnsubstanz nicht erwünscht sein, d.h. eigentlich nur eine Säuberung des Kanallumens vorgenommen werden, so könnte auf eine Diamantierung des Kopfes 13 verzichtet werden. Jedoch kann sowohl in Abhängigkeit von der gewünschten Präparation als auch - wie oben dargestellt - in Abhängigkeit von der verwendeten Frequenz der Einsatz von Diamant-belegten Köpfen von Vorteil sein. Wie aus Fig.1b zu ersehen ist, ist aufgrund der um 90° gebogenen, kurzen Spitze 5 das Einführen derselben auch bei schlechten Sichtverhältnissen erleichtert möglich. Da die Spitze 5 gegen den Schaft 12 um ca. 90° abgewinkelt ist, bietet es sich an - durch eine geeignete Dimensionierung der Länge der Spitze 5 - diesen Winkel als Anschlag bei der Präparation des Wurzelkanals 2 zu nehmen. Markierungsringe 17 (Detail I zu Fig.2b und 2d), die beispielsweise in Millimeterabständen an der Spitze 5 eingraviert sind, erleichtern die Kontrolle über die Präparationstiefe und auch über die Geradeführung der Spitze 5 des Aufbereitungsinstrumentes 4 im Wurzelkanal 2.

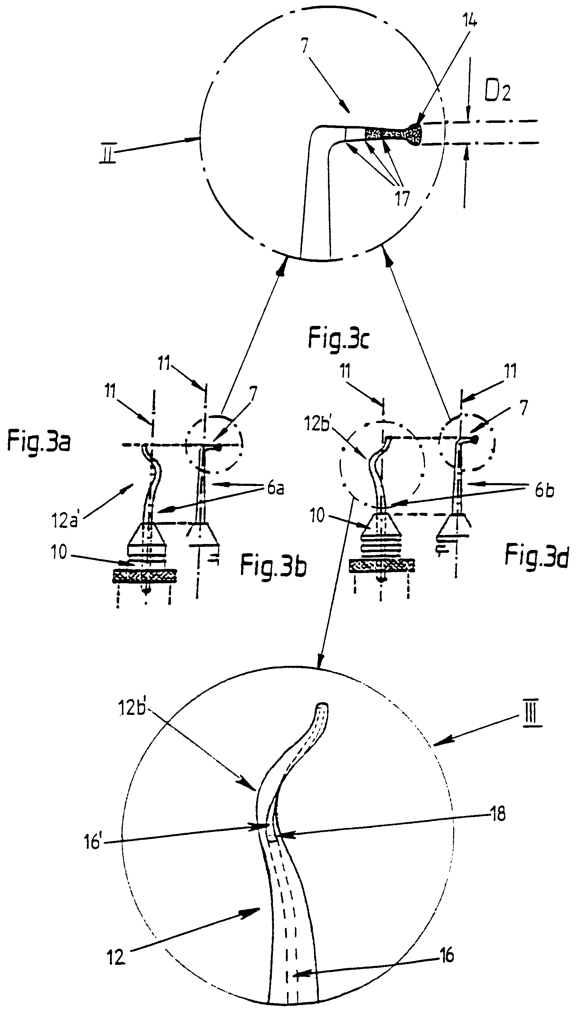

Im nächsten Behandlungsschritt, der in Fig. 1c dargestellt ist, wird ein zweites Aufbereitungsinstrument 6 in den vorpräparierten Wurzelkanal 2 eingebracht. Die Spitze 7 dieses zweiten Aufbereitungsinstrumentes 6 ist ebenfalls um ca. 90° gegen die Erstreckung des Schaftes 12 abgewinkelt, jedoch trägt sie keinen zylindrischen Kopf. Für diesen Präparationsschritt ist ein Aufbereitungsinstrument von Vorteil, dessen Kopf 14, wenn in Schwingungen versetzt, eine Hinterschneidung 8 im Wurzelkanal 2 erzeugt. Ein solcher Kopf 14 kann birnenförmig verdickt ausgebildet sein, wie in Fig.1c dargestellt; aber auch andere ähnliche, beispielsweise ellipsoidförmige, Ausbildungen sind möglich, solange sie eine Hinterschneidung im Wurzelkanal erzeugen. Tropfenförmig ausgebildete Spitzen, wie sie beispielsweise aus der Paradontologie bekannt sind, wären dazu ungeeignet. Der Durchmesser D2 (Detail II aus Fig.3b bzw. 3d) dieses Kopfes 14 an seiner dicksten Stelle und der Durchmesser D1 (Detail I aus Fig.2b bzw. 2d) des Kopfes 13 des ersten Aufbereitungsinstrumentes 4 müssen einander entsprechen, um ein ungehindertes Einführen des zweiten Aufbereitungsinstrumentes 6 in das Wurzelkanallumen zu ermöglichen. D2 darf dementsprechend höchstens gleich gross wie D1 sein.

Die Art und Körnigkeit der Diamantierung auch dieses Kopfes 14 wird sich sowohl nach der gewünschten Präparation richten als auch von der verwendeten Schwingungsfrequenz abhängen, doch wird bei diesem zweiten Aufbereitungsinstrument 6, da immer Zahnbein zu entfernen ist, im allgemeinen eine Diamant-Belegung vorzusehen sein.

Der Knick zwischen Spitze 7 und Schaft 12 dieses zweiten Aufbereitungsinstrumentes 6 kann wieder als Anschlag dienen. Werden noch zusätzlich Markierungsringe 17 (Detail II zu Fig.3b und 3d) an der Spitze 7 vorgesehen, so ist eine erhöhte Sicherheit und erleichterte Führung des Instrumentes bei der Präparation des Wurzelkanals gegeben.

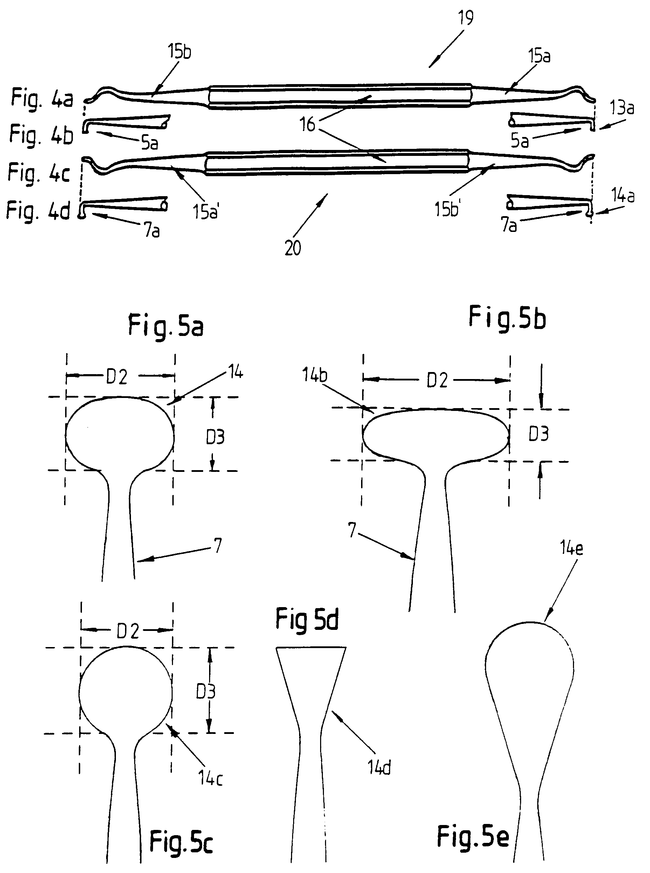

In Fig.1d ist der fertig präparierte Wurzelkanal 2 gezeigt, mit einer unterschnittenen Kavität 9. In diese Kavität 9 wird anschliessend Füllmaterial eingebracht, und komprimiert. Dazu werden die anhand der Fig.4a bis 4d näher beschriebenen Einbringinstrumente benutzt.

In den Fig.2a bis 2d sind zwei Ausbildungsvarianten 4a und 4b für erste Aufbereitungsinstrumente 4 dargestellt, wobei Fig.2a und 2c Ansichten von oben und Fig.2b und 2d Ansichten von der Seite sind. Die Aufbereitungsinstrument 4a und 4b können jeweils in einen Griff 10 gesteckt werden, wobei dieser Griff 10 in den Fig.2a bis 2d nur angedeutet ist. Über diesen an sich - beispielsweise aus der Paradontologie - bekannten Griff 10 werden mechanische Schwingungen, insbesondere Schall- oder Ultraschallschwingungen, einer bestimmten Frequenz auf die Spitze 5 des jeweiligen Aufbereitungsinstrumentes übertragen. Wie aus den Figuren 2b und 2d zu entnehmen ist, besitzen die beiden in den Figuren 2a und 2b, bzw. 2c und 2d dargestellten, ersten Aufbereitungsinstrumente 4a bzw.4b in gleicher Weise eine um 90° gegen die durch Griff 10 und Schaft 12a bzw. 12b des Aufbereitungsinstrumentes 4a bzw. 4b festgelegte Achse 11 gebogene Spitze 5, die jeweils einen zylinderförmigen Kopf 13 trägt. Um den Zugang zu der Behandlungsstelle zu erleichtern, ist dabei, wie aus Fig.2a und 2c zu ersehen ist, der Schaft 12a bzw. 12b des Aufbereitungsinstrumentes 4a bzw. 4b - je nach dem zu behandelnden Quadranten - gebogen ausgebildet, wobei der nach links gebogene Schaft 12a des in Fig.2a dargestellten Aufbereitungsinstrumentes 4a im linken Oberkiefer und im rechten Unterkiefer zur Anwendung kommt. Der nach rechts gebogene Schaft 12b des in Fig.2c dargestellten Aufbereitungsinstrumentes 4b hingegen kommt in analoger Weise bei der Behandlung im rechten Oberkiefer und im linken Unterkiefer zum Einsatz.

Die Figuren 3a bis 3d zeigen in analoger Weise die zweiten Aufbereitungsinstrumente 6a und 6b, die, wie oben beschrieben, eine Hinterschneidung 8 der Kavität 9 im Wurzelkanal 2 (Fig.1c und 1d) bewirken. Fig.3a und 3b zeigen ein solches zweites Aufbereitungsinstrument 6a in Auf- und Seitensicht, dessen Schaft 12a' nach links gebogen und dessen Spitze 7 in bezug auf die Achse 11 um 90° abgebogen ist. Der Kopf 14 an der Spitze 7 ist birnenförmig verdickt, wie in Detail II vergrössert dargestellt, und insbesondere diamantiert. Der Vergleich mit Detail I aus Fig.2b und 2d, das die Ausbildung der Spitze 5 und des Kopfes 13 der ersten Aufbereitungsinstrumente 4a und 4b darstellt, zeigt, dass die beiden Durchmesser D2 und D1 der beiden Köpfe 14 bzw. 13 der beiden Aufbereitungsinstrumente 4 bzw. 6 im wesentlichen gleich gross ist. Jedenfalls sollte der Durchmesser D2 des zweiten Aufbereitungsinstrumentes 6 nicht grösser sein als der Durchmesser D1 des ersten Aufbereitungsinstrumentes 4. Fig.3c und 3d zeigen in analoger Weise ein zweites Aufbereitungsinstrument 6b mit nach rechts gebogenem Schaft 12b'. Die Ausbildung der Spitze 7 entspricht derjenigen des anhand der Fig.3a und 3b beschriebenen Aufbereitungsinstrumentes 6a.

Aus den Details I bzw. II zu den Figuren 2b und 2d bzw. zu 3b und 3d sind die an den Spitzen 5 bzw. 7 angebrachten, insbesondere eingravierten, Markierungsringe 17 zu ersehen. Für die meisten Behandlungsfälle sind Spitzenlängen L von 3 bis 4 mm vorzusehen, die Diamant-Belegung erstreckt sich im allgemeinen über ca. 2mm, und die Markierungsringe 17 können beispielsweise in Millimeterabständen angebracht sein. Insbesondere bei Verwendung des zweiten Aufbereitungsinstrumentes 6 ist der durch den Winkel zwischen Spitze 7 und Schaft 12 definierte Anschlag zur Begrenzung der Eindringtiefe des Aufbereitungsinstrumentes in den Wurzelkanal von grossem Vorteil, da bei zu tiefer Präparierung die Gefahr der Entstehung einer sogenannten "via falsa" gegeben ist.

Je nach zu behandelnder Zahnwurzel werden die Grössen der Durchmesser D1 bzw. D2 des ersten bzw. des zweiten Aufbereitungsinstrumentes 4 bzw. 6 vorzusehen sein. Die Standarddurchmesser werden nach den sogenannten ISO-Normen angegeben. So entspricht der ISO-Norm 80 ein Durchmesser von 0,8 mm, welcher beispielsweise für die Behandlung von Schneidezahn-Wurzeln geeignet ist, und der ISO-Norm 30 ein Durchmesser von 0,3 mm, welcher für die Behandlung von Backenzahn-Wurzeln geeignet ist. Es wird demnach von Vorteil sein, eine Serie von Behandlungsinstrumenten vorzusehen, deren Spitzen unterschiedlich bemasst sind. Praktikabel und auch ausreichend für die meisten Fälle werden Sets sein, die Bahndlungsinstrumente enthalten, deren Kopfdurchmesser 0,5 mm und 0,8 mm betragen, einer IOS-Norm 50 und 80 entsprechend.

Gerade bei den gegebenenfalls mit höheren Frequenzen schwingenden Aufbereitungsinstrumenten ist eine Kühlung während ihres Einsatzes von Vorteil. In diesem Fall wird - wie bereits für andere zahnärztliche Instrumente gebräuchlich - im Inneren des Schaftes des Aufbereitungsinstrumentes ein vorzugsweise bis zur Spitze reichender Kanal für Kühlflüssigkeit vorgesehen werden. Da, wie einleitend dargestellt, die Spitzen sowohl der ersten als auch der zweiten Aufbereitungsinstrumente möglichst fein auszubilden sind, kann gegebenenfalls dieser Kanal nur knapp bis zur Spitze reichen, wobei dann die Kühlflüssigkeit seitlich aus einer Mündungsöffnung austritt und bis zur vibrierenden Spitze hochkriecht. Es kann aber auch, wie aus dem Detail III der Fig.3c zu ersehen ist, der Kanal 16 für die Kühlflüssigkeit nur etwa bis zur Freistellbiegung 12b'im Inneren des Schaftes 12 liegen, aus einer Mündungsöffnung 18 in einen an der Aussenseite des Schaftes 12 ausgefrästen Kanal 16' einströmen, der gewunden, gegebenenfalls etwas spiralförmig, um den Schaft 12 und die Freistellbiegung 12b' vorzugsweise bis wenigstens zur Knickstelle Spitze 7-Schaft 12, dort insbesondere an der Innenseite der Knickstelle endend, verläuft, so dass die dann insbesondere unter Druck und mit einem gewissen Drall austretende Kühlflüssigkeit die restliche Spitze bis zum Kopf 7 entlangkriechen kann.

Die Fig.4a bis 4d zeigen zwei Einbringinstrumente 19 und 20, jeweils mit zwei unterschiedlich gestalteten Schaftenden 15, so dass ein Einbringinstrument durch Wechsel der Griffhaltung am Griff 16 unabhängig vom Quadranten, in dem sich die zu behandelnde Zahnwurzel befindet, eingesetzt werden kann.

Fig.4a und 4b zeigen ein erstes Einbringinstrument 19 in Auf- und Seitensicht. Die Ausbildung der beiden Schaftenden 15a und 15b zu beiden Seiten des Handgriffs 16, sowie der beiden um etwa 90° abgebogenen, einen zylinderförmigen Kopf 13a tragenden Spitzen 5a - selbstverständlich ohne Diamant-Belegung - entspricht den beiden in den Fig.2a bis 2d dargestellten ersten Aufbereitungsinstrumenten 4a und 4b. Dieses erste Einbringinstrument 19 dient dazu, plastisches Wurzelfüllmaterial in den präparierten Wurzelkanal einzubringen.

Das in Fig.4c und 4d in Auf- und Seitensicht dargestellte, zweite Einbringinstrument 20 mit seinen den jeweiligen Ausbildungen der zweiten Aufbereitungsinstrumente 6a und 6b (Fig.3a bis 3d) entsprechend geformten Schaftenden 15a' und 15b' bzw. abgebogenen, birnenförmige Köpfe 14a tragenden Spitzen 7a - wiederum ohne Diamant-Belegung - bewirkt die Komprimierung des bereits im Wurzelkanal plazierten Wurzelfüllmaterials und eine exakte Anlagerung desselben an die Wurzelkanalwandungen.

Konventionelle Einbringinstrumente können gegebenenfalls zusätzlich verwendet werden.

In den Fig. 5a bis 5e sind mögliche Ausbildungsvarianten für an den Spitzen der zweiten Aufbereitungs- bzw. Einbringinstrumente vorzusehende Köpfe dargestellt. Die Darstellung ist nur illustrativ zu sehen, jedoch keineswegs limitierend.

Fig.5a zeigt beispielsweise einen sogenannten "birnenförmigen" Kopf 14, wie bereits in dem Detail II der Fig.3b bzw.3d gezeigt. Der Kopf 14b der Fig.5b könnte als "linsenförmig" oder gegebenenfalls "ellipsoidförmig" bezeichnet werden.

Diesen zwei Köpfen ist gemeinsam, dass der Durchmesser D2 des Kopfes senkrecht zur Längserstreckung der Spitze 7 deutlich grösser ist als der Durchmesser D3 des Kopfes in Richtung der Längserstreckung der Spitze. Wie einfach zu sehen ist, wird dadurch die Führung des Instrumentes im Wurzelkanal bei der Präparation der Hinterschneidung ermöglicht, es entsteht eine "Rille", die Formbestimmung der Hinterschneidung - gerade an dieser unzugänglichen und uneinsehbaren Behandlungsstelle - wird wesentlich erleichtert.

Fig. 5c zeigt einen "Kugelkopf" 14c, die Fig.5d bzw. 5e zeigen zwei alternative Ausbildungen für Köpfe 14d bzw. 14e, die kegelförmig bzw. "tropfenförmig" ausgebildet sind. Werden solche Köpfe verwendet, so werden Kavitäten aufbereitet, deren Durchmesser gegen die Wurzelspitze zu abnimmt. Auf diese Weise ist zwar eine - im Vergleich zu den bekannten Behandlungsmethoden - sicherlich verbesserte Retention des einzubringenden Füllmaterials erzielbar, doch ist in jedem Fall einer Kavität, die eine Hinterschneidung aufweist, aus medizinischen Gründen der Vorzug zu geben.

Es ist ausserdem darauf zu achten, dass der Übergang zwischen Kopf 14 und stabförmiger Spitze 7 nicht scharfkantig ist. Nicht nur, dass damit die Präparation diffiziler wird - es besteht die Gefahr Zahmaterial abzureissen - auch ist nach erfolgter Präparierung das Füllmaterial nicht so gut zu befestigen, es könnten Hohlräume entstehen, die nicht gut abgefüllt sind.

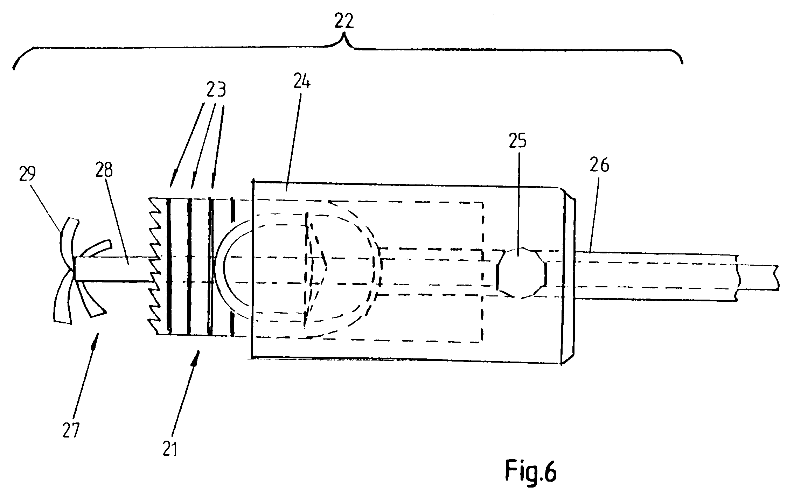

In Fig.6 ist ein Knochendeckelaufbereitungsinstrument 22 gezeigt, das einen Trepanationsbohrer 21 aufweist, der als Hohlzylinder mit Stirnverzahnung ausgebildet ist. Markierungen 23 an der Aussenseite des Hohlzylinders ermöglichen die Kontrolle über die jeweilige Eindringtiefe. Ein diesen Hohlzylinder umgebender Anschlagszylinder 24 kann mittels einer Stellschraube 25 auf die gewünschte, maximale Eindringtiefe eingestellt werden. Kühlflüssigkeit für den Trepanationsbohrer 21 wird vorzugsweise intern zugeführt, gegebenenfalls über einen im Schaft 26 vorgesehenen Kanal (nicht dargestellt). Ein Retentions- und Repositionselment 27, das vorzugsweise im Inneren des Schaftes 26 des Trepanationsbohrers 21 geführt ist, ist beispielweise in Form eines vorzugsweise zylindrischen Aspirationsstempels einer Carpulenspritze ausgebildet. Ein Stab 28 wird durch ein im Knochendeckel ausgefrästes Loch geführt, wobei dieses Loch durch den gegebenenfalls gleichzeitig als Bohrer ausgebildeten und über den gleichen Motor wie der Trepanationsbohrer 21 in Rotation versetzbaren Stab 28 erzielbar ist. Werkzeugmassig einfacher wird es allerdings sein, wenn ein solches Loch unabhängig vorgebohrt, der Stab 28 als Justier- und Zentrierhilfe in diese Loch eingesteckt wird und danach der Knochendeckel gewünschter Dicke ausgefräst wird. Dann kann der Stab 28 durch diesen Knochendeckel durchgesteckt werden, ein sternartiges Gebilde 29 wird ausgefaltet und der Knochendeckel kann - ohne den durch den Trepanationsbohrer 21 ausgefrästen Rand zu verletzen - abgezogen werden. Der Knochendeckel kann innerhalb des Hohlzylinders des Trepanationsbohrers 21 gehalten werden und nach erfolgter Behandlung der Zahnwurzel wieder eingesetzt werden. Der korrekt eingestellte Anschlagszylinder erleichtert dabei das positionsgetreue Wiedereinsetzen des Knochendeckels in den Kiefer. Das sternartige Gebilde 29, das nicht nur das Lösen des Knochendeckels sondern auch die Retention desselben innerhalb des Hohlzylinders des Trepanationsbohrers 21 ermöglicht, wird dann in den Stab 28 eingezogen, dieser wird aus dem zentralen Loch zurückgezogen, das sternartige Gebilde 29 wird wieder entfaltet, und hilft so bei der Repositionierung des Knochendeckels mit.

Ein solches Retentions- und Repositionselement könnte auch lateral im Trepanationsbohrer angeordnet sein, auch ein nach dem Ausfräsen des Knochendeckels unabhängig von diesem arbeitendes, entsprechend ausgebildetes Instrument ist möglich.

## Patentansprüche

1. Zahnbehandlungsinstrument zur retrograden Behandlung von Wurzelkanälen (2), mit wenigstens einem mit einem Handgriff (10;16) verbundenen, länglichen, in einer Spitze (7;7a) endenden Schaft (12;15), wobei die Spitze (7;7a) gegenüber einer durch die Längserstreckung des Schaftes (12;15) bzw. des Handgriffes (10;16) definierten Achse (11) um etwa 90° abgebogen ist, dadurch gekennzeichnet, dass ein am freien Ende der Spitze (7;7a) vorgesehener, insbesondere rotationssymmetrischer, Kopf (14; 14a;...;14e) zum achsengerechten Aufbereiten einer eine Hinterschneidung (8) aufweisenden Kavität (9) im Wurzelspitzenbereich, deren Durchmesser gegen die Wurzelspitze (3) zu geringer ist, bzw. zum Einbringen von Füllmaterial in eben diese Kavität (9) ausgebildet ist, wobei - im Querschnitt gesehen - der Durchmesser (D2) des Kopfes (14;14a;14b) senkrecht zur Längserstreckung der Spitze (7;7a) gemessen, grösser als der Durchmesser (D3) des Kopfes (14;14a;14b) in Richtung der Längserstreckung der Spitze (7) gemessen - insbesondere um wenigstens 25% grösser - ist, und vorzugsweise in Form eines - wenigstens an der der Spitze zugewandten Seite im wesentlichen platten - wulstförmigen Flansches, oder in Form einer auf dem freien Ende der Spitze aufsitzenden, abgerundeten Linse oder in Form einer Birne ausgebildet ist.

2. Zahnbehandlungsinstrument nach Anspruch 1, dadurch gekennzeichnet, dass der Schaft (12;15) eine in bezug auf die Achse (11) nach rechts oder nach links weisende Freistellungsbiegung (12a';12b') aufweist, jeweils einer rechts- oder linkshändigen Ausbildung des Zahnbehandlungsinstrumentes (6a,6b; 20) entsprechend.

3. Zahnbehandlungsinstrument nach einem der vorhergehenden Ansprüche, dadurch gekennzeichnet, dass an der Spitze (7) wenigstens eine Markierung (17) zur Kontrolle der Behandlungstiefe vorgesehen ist.

4. Zahnbehandlungsinstrument nach einem der vorhergehenden Ansprüche, dadurch gekennzeichnet, dass der Kopf (14) eine Diamant-Belegung aufweist.

5. Zahnbehandlungsinstrument nach einem der vorhergehenden Ansprüche, dadurch gekennzeichnet, dass im Inneren des Schaftes (12) bzw. wenigstens teilweise am Schaft (12) - vorzugsweise bis zum Kopf(14) reichend - ein Kanal (16,16') zum Transport von Kühlflüssigkeit bis zum Behandlungsort vorgesehen ist.

6. Zahnbehandlungsinstrument nach Anspruch 5, dadurch gekennzeichnet, dass der Kanal (16') an der Aussenseite des Schaftes (12), und gegebenenfalls der Spitze (7), insbesondere wenigstens teilweise spiralförmig verlaufend ausgebildet ist.

7. Zahnbehandlungsinstrument nach einem der vorhergehenden Ansprüche, zur Verwendung als Aufbereitungsinstrument (5), dadurch gekennzeichnet, dass es - vorzugsweise mittels eines Schall- oder Ultraschallschwingungen erzeugenden oder übertragenden Griffes (10) - in Schwingungen versetzbar ist.

8. Zahnbehandlungsinstrument nach einem der Ansprüche 1 bis 3, zur Verwendung als Einbringinstrument (20), dadurch gekennzeichnet, dass es an einem Handgriff (16) zwei unterschiedlich ausgebildete Schaftenden (15a',15b') aufweist, insbesondere jeweils eine rechts- und linkshändige Variante mit jeweils gleicher Kopfform.

9. Set von Zahnbehandlungsinstrumenten mit jeweils wenigstens einem zweiten Aufbereitungsinstrument (6) nach Anspruch 7 und wenigstens einem zweiten Einbringinstrument (20) nach Anspruch 8, sowie jeweils wenigstens einem in Schwingungen versetzbaren, ersten Aufbereitungsinstrument (4 ) und einem ersten Einbringinstrument (19), deren Schaft- und Spitzenausbildung jeweils dem in dem Set vorliegenden, zweiten Aufbereitungs- (6) bzw. Einbringinstrument (20) entpricht, die Form des Kopfes (13;13a) jedoch zylinderförmig ausgebildet ist.

10. Set von Behandlungsinstrumenten nach Anspruch 9, dadurch gekennzeichnet, dass es zusätzlich einen Schall- oder Ultraschallschwingungen erzeugenden oder übertragenden Griff (10) enthält, in den das erste (4) bzw. das zweite Aufbereitungsinstrument (6) einsteckbar ist.

11. Set von Behandlungsinstrumenten nach Anspruch 9 oder 10, dadurch gekennzeichnet, dass der Kopf (13) des ersten Aufbereitungsinstrumentes (6) eine Diamant-Belegung aufweist, deren Korngrösse insbesondere derjenigen der Diamant-Belegung des zweiten Aufbereitungsinstrumentes (6) entspricht.

12. Set von Behandlungsinstrumenten nach einem der Ansprüche 9 bis 11, dadurch gekennzeichnet, dass die Durchmesser (D2;D1) der Köpfe (14;13) jeweils eines Paares aus einem zweiten (6) und einem ersten (4) Aufbereitungsinstrument - senkrecht zur Längserstreckung der Spitze (7;5) gemessen - im wesentlichen gleich gross sind, und dass vorzugsweise auch die Durchmesser der Köpfe (14a;13a) jeweils eines Paares aus einem zweiten (20) und einem ersten (19) Einbringinstrument - ebenfalls senkrecht zur Längserstreckung der Spitze (7a;5a) gemessen - im wesentlichen gleich gross und insbesondere geringfügig kleiner als der Durchmesser der Köpfe (7;5) der Aufbereitungsinstrumente (6;4) ist.

13. Set von Zahnbehandlungsinstrumenten nach einem der Ansprüche 9 bis 12, dadurch gekennzeichnet, dass es wenigstens zwei gleichartige, jedoch unterschiedliche Kopfdurchmesser (D1;D2) aufweisende Aufbereitungsinstrumente (4;6) und wenigstens zwei gleichartige Einbringinstrumente (19;20) beinhaltet, wobei deren Köpfe unterschiedliche Durchmesser (D1,D2) aufweisen.

14. Set von Zahnbehandlungsinstrumenten nach einem der Ansprüche 9 bis 13, dadurch gekennzeichnet, dass es zusätzlich ein Knochendeckelaufbereitungsinstrument (22) enthält, das einen Trepanationsbohrer (21) aufweist.

15. Set von Zahnbehandlungsinstrumenten nach Anspruch 14, dadurch gekennzeichnet, dass dem Knochendeckelaufbereitungsinstrument (22) ein Retentions- und/oder Repositionselement (27), insbesondere als integrierter Bestandteil, zugeordnet ist.

16. Set von Zahnbehandlungsinstrumenten nach Anspruch 14 oder 15, dadurch gekennzeichnet, dass der Hohlzylinder des Trepanationsbohrers (21) Markierungen (23) zur Bestimmung der Eindringtiefe und gegebenenfalls einen Anschlag (24) zur Einstellung einer vorgegebenen, maximalen Eindringtiefe aufweist.

17. Set von Zahnbehandlungsinstrumenten nach Anspruch 15 oder 16, das das Retentions- und/oder das Repositionselement (27) einen - vorzugsweise im Zentrum des Trepanationsbohrers (21) angeordneten - gegebenenfalls als Bohrer ausgebildeten Führungsstab (28) mit einem die Retention bzw. die Reposition des Knochendeckeis bewirkenden aus- bzw. einführbaren, gegebenenfalls sternartigen, Gebildes (29) aufweist.

## Claims

1. Dental treatment instrument for retrograde treatment of root canals (2), having at least one elongated shaft (12; 15) connected to a handle (10; 16) and ending in a point (7; 7a), the point (7; 7a) being bent by about 90° relative to an axis (11) defined by the longitudinal dimension of the shaft (12; 15) or of the handle (10; 16), characterized in that a head (14; 14a; ...; 14e) which is provided at the free end of the point (7; 7a) and is in particular rotationally symmetrical is formed for correct axial preparation of a cavity (9) in the root apex region, which cavity has an undercut (8) and whose diameter is smaller than the root apex (3), or for introducing filling materials into this cavity (9), the diameter (D2) of the head (14; 14a; 14b), viewed in cross-section and measured at right angles to the longitudinal dimension of the point (7; 7a), being larger than the diameter (D3) of the head (14; 14a; 14b) measured in the direction of the longitudinal dimension of the point (7), in particular larger by at least 25%, and preferably being in the form of a bead-like flange which is essentially flat at least on the side facing the point, or being in the form of a rounded lens resting on the free end of the point, or being in the form of a pear.

2. Dental treatment instrument according to Claim 1, characterized in that the shaft (12; 15) has a space-providing curve (12a'; 12b') pointing to the right or to the left relative to the axis (11), in each case according to a right- or left-handed form of the dental treatment instrument (6a; 6b; 20).

3. Dental treatment instrument according to any of the preceding Claims, characterized in that at least one mark (17) for checking the treatment depth is provided on the point (7).

4. Dental treatment Instrument according to any of the preceding Claims, characterized in that the head (14) has a diamond coating.

5. Dental treatment instrument according to any of the preceding Claims, characterized in that a channel (16, 16') for transporting cooling liquid to the place of treatment is provided in the interior of the shaft (12) or at least partly on the shaft (12) - preferably extending up to the head (14).

6. Dental treatment instrument according to Claim 5, characterized in that the channel (16') is formed on the outside of the shaft (12) and optionally of the point (7), in particular at least partly in spiral form.

7. Dental treatment instrument according to any of the preceding Claims, for use as a preparation instrument (5), characterized in that it can be caused to oscillate, preferably by means of a handle (10) which generates or transmits sonic or ultrasonic oscillations.

8. Dental treatment instrument according to any of Claims 1 to 3, for use as an insertion instrument (20), characterized in that it has, on a handle (16), two differently formed shaft ends (15a', 15b'), in particular a right- and left-handed variant, each having the same head shape.

9. Set of dental treatment instruments having in each case at least one second preparation instrument (6) according to Claim 7 and at least one second insertion instrument (20) according to Claim 8, and in each case at least one first preparation instrument (4) which can be caused to oscillate and one first insertion instrument (19) whose shaft and point form correspond in each case to the second preparation instrument (6) or insertion instrument (20) present in this set, but the shape of the head (13; 13a) being cylindrical.

10. Set of treatment instruments according to Claim 9, characterized in that it additionally contains a handle (10) which generates or transmits sonic or ultrasonic oscillations and into which the first (4) or the second preparation instrument (6) can be inserted.

11. Set of treatment instruments according to Claim 9 or 10, characterized in that the head (13) of the first preparation instrument (6) has a diamond coating whose particle size corresponds in particular to the diamond coating of the second preparation instrument (6).

12. Set of treatment instruments according to any of Claims 9 to 11, characterized in that the diameters (D2; D1) of the heads (14; 13) of each pair comprising a second (6) and a first (4) preparation instrument are essentially the same size, measured at right angles to the longitudinal dimension of the point (7; 5), and that preferably also the diameters of the heads (14a; 13a) of each pair comprising a second (20) and a first (19) insertion instrument, also measured at right angles to the longitudinal dimension of the point (7a; 5a), are essentially the same size and in particular slightly smaller than the diameters of the heads (7; 5) of the preparation instruments (6; 4).

13. Set of dental treatment instruments according to any of Claims 9 to 12, characterized in that it contains at least two preparation instruments (4; 6) which are of the same type but have different head diameters (D1; D2) and at least two insertion instruments (19; 20) of the same type, their heads having different diameters (D1, D2).

14. Set of dental treatment instruments according to any of Claims 9 to 13, characterized in that it additionally contains a bone cover preparation instrument (22) which has a trepanation drill (21).

15. Set of dental treatment instruments according to Claim 14, characterized in that a retainer and/or setter (27) is coordinated with the bone cover preparation instrument (22), in particular as an integral part.

16. Set of dental treatment instruments according to Claim 14 or 15, characterized in that the hollow cylinder of the trepanation drill (21) has marks (23) for determining the depth of penetration and optionally a stop (24) for setting a predetermined, maximum depth of penetration.

17. Set of dental treatment instruments according to Claim 15 or 16, characterized in that the retainer and/or the setter (27) has a guide rod (28) which is preferably arranged in the centre of the trepanation drill (21), is optionally in the form of a drill and has an extendable and retractable, optionally star-shaped structure (29) which implements the retention or the setting of the bone cover.

## Revendications

1. Instrument de soins dentaires pour le traitement rétrograde de canaux radiculaires (2), comportant au moins une tige (12; 15) allongée, s'achevant en une pointe (7; 7a), reliée à une poignée (10; 16), la pointe (7; 7a) étant repliée d'environ 90° par rapport à un axe (11) défini par la direction longitudinale de la tige (12; 15) ou de la poignée (10; 16), caractérisé en ce qu'une tête (14; 14a; ...; 14e), prévue à l'extrémité libre de la pointe (7; 7a), en particulier répondant à une symétrie de rotation, est réalisée pour la préparation, dans le bon axe, d'une cavité (9) présentant une contre-dépouille (8) dans la zone de la pointe de racine, dont le diamètre est trop faible par rapport à la pointe de racine (3), respectivement pour introduire un matériau de remplissage justement dans cette cavité (9), le diamètre (D2) - lorsque l'on observe en coupe transversale - de la tête (14; 14a; 14b), mesuré perpendiculairement par rapport à la direction longitudinale de la pointe (7; 7a), étant supérieur au diamètre (D3) de la tête (14; 14a; 14b) mesuré dans la direction de la direction longitudinale de la pointe (7) - en particulier supérieur d'au moins 25 % - et de préférence étant réalisé sous la forme d'un rebord en forme de bourrelet - au moins sensiblement plat sur la face tournée vers la pointe -, ou bien sous la forme d'une lentille arrondie siégeant sur l'extrémité libre de la pointe ou bien sous la forme d'une poire.

2. Instrument de soins dentaires selon la revendication 1, caractérisé en ce que la tige (12, 15) présente un pliage de position libre (12a'; 12b') tourné vers la droite ou vers la gauche par rapport à l'axe (11), correspondant respectivement à une réalisation pour droitier ou gaucher de l'instrument de soins dentaires (6a, 6b; 20).

3. Instrument de soins dentaires selon l'une des revendications précédentes, caractérisé en ce qu'au moins un marquage (17) est prévu sur la pointe (7), pour assurer le contrôle de la profondeur de traitement.

4. Instrument de soins dentaires selon l'une des revendications précédentes, caractérisé en ce que la tête (14) présente un garnissage en diamant.

5. Instrument de soins dentaires selon l'une des revendications précédentes, caractérisé en ce qu'à l'intérieur de la tige (12), respectivement au moins partiellement sur la tige (12), - en allant de préférence jusqu'à la tête (14) - est prévu un canal (16, 16') destiné au transport jusqu'au site de traitement d'un liquide de refroidissement.

6. Instrument de soins dentaires selon la revendication 5, caractérisé en ce que le canal (16') est réalisé sur la face extérieure de la tige (12) et, le cas échéant, de la pointe (7), en s'étendant en particulier au moins partiellement en forme de spirale.

7. Instrument de soins dentaires selon l'une des revendications précédentes, pour utilisation comme instrument de préparation (5), caractérisé en ce qu'il est susceptible d'être mis en vibration - de préférence au moyen d'un manche (10) générant ou transmettant des vibrations sonores ou ultrasonores.

8. Instrument de soins dentaires selon l'une des revendications 1 à 3 pour utilisation comme instrument d'introduction (20), caractérisé en ce qu'il présente sur une poignée (16) deux extrémités de tige (15a', 15b') de réalisation différente, ayant en particulier chacune une variante pour droitier et gaucher, avec chaque fois la même forme de tête.

9. Jeu d'instruments de soins dentaires ayant respectivement au moins un deuxième instrument de préparation (6) selon la revendication 7 et au moins un deuxième instrument d'introduction (20) selon la revendication 8, ainsi que respectivement au moins un premier instrument de préparation (4) susceptible d'être mis en vibration et un premier instrument d'introduction (19), dont la réalisation de la tige et de la pointe correspond respectivement au deuxième instrument de préparation (6) ou d'introduction (20) présent dans le jeu, la forme de la tête (13; 13a) étant cependant cylindrique.

10. Jeu d'instruments de traitement selon la revendication 9, caractérisé en ce qu'il contient en plus un manche (10) générant ou transmettant des vibrations sonores ou ultrasonores, manche dans lequel le premier (4) ou le deuxième (6) instrument de préparation est susceptible d'être enfiché.

11. Jeu d'instruments de traitement selon la revendication 9 ou 10, caractérisé en ce que la tête (13) du premier instrument de préparation (6) présente un garnissage en diamant, dont la taille de grains correspond en particulier à celle du garnissage en diamant du deuxième instrument de préparation (6).

12. Jeu d'instruments de traitement selon l'une des revendications 9 à 11, caractérisé en ce que les diamètres (D2; D1) des têtes (14; 13) d'une paire respective constituée d'un deuxième (6) et d'un premier (4) instrument de préparation sont - lorsque l'on mesure perpendiculairement par rapport à la direction longitudinale de la pointe (7; 5) - sensiblement de même valeur, et en ce que, de préférence, également les diamètres des têtes (14a; 12a), respectivement d'une paire constituée d'un deuxième (20) et d'un premier (19) instrument d'introduction, sont - lorsque l'on mesure également perpendiculairement par rapport à la direction longitudinale des pointes (7a; 5a) - sensiblement de la même valeur et en particulier légèrement inférieurs aux diamètres des têtes (7; 5) des instruments de préparation (6; 4).

13. Jeu d'instruments de soins dentaires selon l'une des revendications 9 à 12, caractérisé en ce qu'il contient au moins deux instruments de préparation (4; 6) de même type, cependant d'un diamètre de tête (D1; D2) différent, et au moins deux instruments d'introduction (19; 20) de même type, leurs têtes présentant des diamètres (D1, D2) différents.

14. Jeu d'instruments de soins dentaires selon l'une des revendications 9 à 13, caractérisé en ce qu'il contient en plus un instrument de préparation du couvercle osseux (22), présentant une fraise de trépanation (21).

15. Jeu d'instruments de soins dentaires selon la revendication 14, caractérisé en ce qu'est associé à l'instrument de préparation du couvercle osseux (22) un élément de rétention et/ou de repositionnement (27), en particulier sous la forme d'un composant intégré.

16. Jeu d'instruments de soins dentaires selon la revendication 14 ou 15, caractérisé en ce que le cylindre creux de la fraise de trépanation (21) présente des marquages (23) destinés a déterminer la profondeur de pénétration et, le cas échéant, une butée (24) destinée à régler une profondeur de pénétration maximale, prédéterminée.

17. Jeu d'instruments de soins dentaires selon la revendication 15 ou 16, caractérisé en ce que l'élément de rétention et/ou de repositionnement (27) présente une barre de guidage (28) - disposée de préférence au centre de la fraise de trépanation (21) - le cas échéant réalisée sous forme de fraise, avec une structure (29) provoquant la rétention ou le repositionnement du couvercle osseux, structure pouvant être guidée en sortie ou en pénétration, le cas échéant du genre d'une étoile.
